# EUROPEAN PATENT SPECIFICATION

(11) **EP 4 180 552 B1**
(45) Date of publication and mention of the grant of the patent: **20.05.2026**
(21) Application number: 21847092.0
(22) Date of filing: 22.06.2021
(51) Int. Cl.: A61L 31/02, A61L 31/08, A61L 27/04, A61L 27/30, C25D 7/04, C23C 30/00, A61F 2/89, A61F 2/915

(54) **IMPLANTABLE MEDICAL DEVICE AND METHOD FOR MANUFACTURING SAME**
IMPLANTIERBARE MEDIZINISCHE VORRICHTUNG UND VERFAHREN ZUR HERSTELLUNG DAVON
DISPOSITIF MÉDICAL IMPLANTABLE ET SON PROCÉDÉ DE FABRICATION

(30) Priority: 22.07.2020 CN 202010711084
(43) Date of publication of application: 17.05.2023
(73) Proprietor: Shanghai MicroPort Medical (Group) Co., Ltd., Shanghai 201203 (CN)
(72) Inventor: WANG, Jiaojiao, Shanghai 201203 (CN); ZHOU, Xinyu, Shanghai 201203 (CN); YAO, Yao, Shanghai 201203 (CN); ZHOU, Qi, Shanghai 201203 (CN); LI, Junfei, Shanghai 201203 (CN)
(74) Representative: Arnold & Siedsma
(86) International application number: PCT/CN2021/101453
(87) International publication number: WO 2022/017101

(56) References cited:
- EP-A2- 1 290 984
- CN-A- 1 250 820
- CN-A- 101 476 108
- CN-A- 104 178 765
- CN-A- 111 632 205
- CN-A- 112 111 738
- CN-A- 113 230 462
- US-A1- 2016 122 865

## Description

### TECHNICAL FIELD

The present disclosure relates to the field of medical technology, and particularly to an implantable medical device and a method for manufacturing thereof

### BACKGROUND

Medical metal materials are one of the largest categories of the most widely used biomedical materials in clinical application due to their excellent and comprehensive mechanical properties, excellent corrosion resistance, good biocompatibility, and excellent processing performance. Currently, medical metal materials are mainly used in the manufacture of implantable medical devices such as internal implants and artificial prostheses in orthopedic, dental and orthopedic surgery. Typical representative of medical metal materials are various luminal stents such as cardiovascular stents and various surgical instruments and tools, which play an important role in prolonging the life time of a patient, improving the function of the affected limb, improving the overall life quality of a patient, and the like. EP 1 290 984 A2 relates to a protective coating for a stent with intermediate radiopaque coating. US 2016/122865 A1 relates to a process for the manufacture of a thin-film multilayered coating used in treating biomedical substrates and a coating in multilayered thin-film form to treat biomedical substrates used in surgical implants. CN104178765A relates to biomedical metal implant materials or interventional technologies, and in particular to a method for preparing a controllable degradation metal composite coating on a surface of a medical magnesium alloy. CN1250820A relates to a porous titanium biocoated bone implant and manufacturing method thereof.

The medical metal materials currently used in the clinical application mainly include stainless steel, cobalt-based alloys and titanium alloys. The total use amount of these medical metal materials accounts for more than 40% of the implantable medical device materials, which play an important role in the production and clinical application of the medical metal materials. However, all of these medical metal materials contain nickel, but it has been found that nickel ions are cytotoxic, teratogenic and carcinogenic, which may result in a biological safety problem due to dissolution of nickel ions after implantation of a stent in a human body. In addition, cobalt ions in cobalt-based alloys are currently considered to be cytotoxic and also have biological safety concerns.

### SUMMARY

According to various embodiments of the present disclosure, an implantable medical device and a manufacturing method thereof are provided.

According to the invention , an implantable medical device is provided. The implantable medical device includes a metal substrate and a cladding layer; wherein the cladding layer is a nickel-free and cobalt-free metal layer and is deposited on the metal substrate by metal bonding. The implantable medical device further comprises at least one reinforcing layer deposited between the metal substrate and the cladding layer by metal bonding; wherein a material of the reinforcing layer is selected from at least one of a metal element and a metal alloy; wherein the cladding layer or/and the reinforcing layer contain(s) hydroxyl groups.

According to one embodiment, a density of the cladding layer is greater than that of the metal substrate.

According to one embodiment, a thickness of the cladding layer is less than or equal to 25 µm.

According to one embodiment, the material of the cladding layer is selected from at least one of a metal element or a metal alloy; wherein the metal element is Ir, Pt, Ru, Ta, W or Au; and the metal alloy includes at least one of PtIr, PtPd, or PtRh.

According to the invention, the implantable medical device further comprises at least one reinforcing layer deposited between the metal substrate and the cladding layer by metal bonding.

According to one embodiment, a total thickness of the reinforcing layer is less than or equal to 500 nm.

According to one embodiment, a material of the reinforcing layer is selected from at least one of a metal element, a metal alloy, a metal oxide, or a metal nitride.

According to one embodiment, the metal element is Ir, Pt, Ru, Ta, W or Au; the metal alloy includes at least one of PtIr, PtPd, or PtRh; the metal oxide includes at least one of HfO₂, TiO₂, Ta₂O₅, ZnO, ZrO₂, MgO, SrTiOₓ, La₂O₃, or CeO₂; and the metal nitride includes at least one of TiN or TaNx.

According to the invention, the cladding layer or/and the reinforcing layer contain(s) hydroxyl groups.

According to one embodiment, a mass fraction of the hydroxyl groups is less than or equal to 0.5%.

According to another aspect of the invention, a method for manufacturing the implantable medical device of any one of the above embodiments is provided. The method for manufacturing the implantable medical device includes: forming a metal substrate; and depositing a cladding layer on the metal substrate by metal bonding, wherein the cladding layer is a nickel-free and cobalt-free metal layer.

According to one embodiment, the forming a metal substrate includes cutting a target material to obtain the metal substrate.

According to the invention, the method further includes depositing at least one reinforcing layer on the metal substrate by metal bonding prior to depositing a cladding layer on the metal substrate by metal bonding.

According to one embodiment, the cladding layer or/and the reinforcing layer are deposited by at least one of physical vapor deposition, thermal spraying, pulsed laser thin film deposition, magnetron sputtering deposition, evaporative plating, ion plating, electroless plating, electrochemical plating, chemical vapor deposition, anodic oxidation, ion implantation and deposition, or glow discharge plasma treatment.

According to one embodiment, the method further includes forming a groove on the metal substrate prior to the depositing a cladding layer on the metal substrate by metal bonding.

Further disclosed but not claimed is a method for manufacturing a vascular stent which includes: cutting a metal tube to form a stent substrate having a supporting rod; and depositing a cladding layer on the stent substrate by metal-bonding, wherein the cladding layer is a nickel-free and cobalt-free metal layer.

According to one embodiment, the method further includes depositing at least one reinforcing layer on the stent substrate by metal bonding prior to the depositing a cladding layer on the stent substrate by metal bonding.

According to one embodiment, the method further includes forming a groove on the metal substrate prior to the depositing a cladding layer on the stent substrate by metal bonding.

According to one embodiment, the cladding layer and/or the reinforcing layer contain(s) hydroxyl groups.

In the above implantable medical device, the method for manufacturing thereof, and the method for manufacturing a vascular stent, the cladding layer is provided on a metal substrate (equivalent to a stent substrate) to prevent dissolution of sensitization and carcinogenic elements such as nickel and cobalt from the metal substrate. In addition, since the cladding layer itself is a nickel-free and cobalt-free metal layer, there is no dissolution of nickel or cobalt from the whole implantable medical device, thereby ensuring the biological safety of the implantable medical device and further improving the biocompatibility of the implantable medical device. In addition, when the cladding layer is configured as the nickel-free and cobalt-free metal layer, the cladding layer itself can be deposited on the metal substrate by metal bonding, thereby effectively preventing the cladding layer from cracking or even peeling off. For example, when the implantable medical device is used as a vascular stent, the cladding layer will not be cracked or peeled off after the vascular stent is crimped and extremely dilated at least by 30% of the cold deformation amount. This can not only completely avoid dissolution of sensitization and oncogenic elements such as nickel and cobalt from the metal substrate, but also can improve corrosion resistance of the whole implantable medical device in a patient's body, thereby avoiding undesirable effects on the patient's health. For example, when the implantable medical device is used as a vascular stent, serious accidents such as thrombotic death due to peeling of the cladding layer can be avoided. It should be noted that, if the metal substrate is directly configured as a nickel-free and cobalt-free metal substrate, it would be subjected to a series of procedures such as composition optimization design, experimental verification, ingot smelting, solution heat treatment, forging, rolling, annealing, tube blank processing, tube drawing, and stress relieving annealing, making the entire process have complicated procedures, a long processing period, a large investment, and high risks. However, in the embodiments of the present disclosure, the nickel-free and cobalt-free cladding layer is provided on the metal substrate by depositing, which is equivalent to modifying the surface of the metal substrate, thus it is not necessary to perform the above procedures, the processing procedures of the implantable medical device can be simplified, the processing period of the implantable medical device can be shortened, and the processing cost of the implantable medical device can be reduced.

### BRIEF DESCRIPTION OF THE DRAWINGS

The drawings forming part of the present disclosure are used to provide further understanding of the present disclosure, and the illustrative embodiments and descriptions of the present disclosure are used to explain the present disclosure and not to be construed as unduly limiting the present disclosure.

In order to illustrate technical solutions in the embodiments of the present disclosure more clearly, the drawings used in the embodiments will be described briefly. Apparently, the following described drawings are merely for some embodiments of the present disclosure, and other drawings can be derived by those of ordinary skill in the art without any creative effort.
FIG. 1 is a schematic view of the structure of an implantable medical device used as a vascular stent according to one embodiment of the present disclosure.
FIG. 2 is a schematic view of the partial structure of an implantable medical device used as a vascular stent according to one embodiment of the present disclosure.
FIG. 3 is a schematic cross-sectional view of the structure of an implantable medical device taken along line A-A in FIG. 2 according to one embodiment of the present disclosure.
FIG. 4 is a schematic cross-sectional view of the structure of an implantable medical device taken along line A-A in FIG. 2 according to another embodiment of the present disclosure.
FIG. 5 is a schematic view of the partial structure of an implantable medical device used as a vascular stent according to another embodiment of the present disclosure.
FIG. 6 is a schematic cross-sectional view of the structure of an implantable medical device taken along line A-A in FIG. 5 according to another embodiment of the present disclosure.
FIG. 7 is a schematic cross-sectional view of an implantable medical device taken along line A-A in FIG. 5 according to another embodiment of the present disclosure.
FIG. 8 is a flow chart of a process for manufacturing an implantable medical device according to another embodiment of the present disclosure.
FIG. 9 is a flow chart of a process for manufacturing a vascular stent according to another embodiment of the present disclosure.
FIG. 10 (a) is a scanning electron microscope view of a reinforcing ring site of an implantable medical device prior to extreme dilation according to one embodiment of the present disclosure.
FIGS. 10 (b) to 10 (g) are scanning electron microscopic views of a reinforcing ring site of an implantable medical device after extreme dilation according to one embodiment of the present disclosure.
FIG. 11 (b) is a scanning electron microscope view of the implantable medical device having a drug-carrying slot after extreme dilation according to one embodiment of the present disclosure.
FIG. 11 (c) is a partial scanning electron microscope view of the drug-carrying slot of the implantable medical device after extreme dilation according to one embodiment of the present disclosure.

### DETAILED DESCRIPTION OF THE EMBODIMENTS

In order to make the above purposes, features and advantages of the present disclosure more apparent, the specific embodiments of the present disclosure are described in detail below with reference to the drawings. Many specific details are set forth in the following description to facilitate a full understanding of the present disclosure. However, the present disclosure can be implemented in many other ways different from those described herein, and those skilled in the art can make similar improvements without departing the concept of the present disclosure, and thus the present disclosure is not limited by the specific embodiments disclosed below.

In the description of the present disclosure, it is necessary to understand that the terms "center," "longitudinal," "lateral," "length," "width," "thickness," "above," "below," "front," "back," "left," "right," "vertical," "horizontal," "top," "bottom," "inner," "outer," "clockwise," "counterclockwise," "axial," "radial," "circumferential" and the like indicated orientation or position relationships are based on the orientation or position relationships shown in the drawings solely for the purpose of describing the present disclosure and simplifying the description, rather than indicating or implying that the device or element indicated must have a specific orientation, be constructed and operated with a specific orientation, and therefore could not be considered as a restriction on the present disclosure.

In addition, the terms "first" and "second" are used for descriptive purposes only and could not be considered as indicating or implying relative importance or implied indication of the number of technical features indicated. Thus, a feature defined as "first" or "second" may expressly or implicitly include at least one such feature. In the description of the present disclosure, "plurality of" means at least two, such as two, three, etc., unless otherwise specifically indicated.

In the present disclosure, unless otherwise specifically stated and defined, the terms "install," "attach," "connect," "fix", and the like shall be understood broadly, for example, can be a fixed attachment, can be a detachable attachment, or can be integrated; can be mechanical connection, or can be electrical connection; can be directly connected or indirectly connected via an intermediate medium; and can be a connection within two elements or an interaction between two elements, unless otherwise specifically defined. For those skilled in the art, the specific meanings of the above terms in the present disclosure can be understood according to the specific context.

In the present disclosure, unless otherwise specifically stated and defined, a first feature is "above" or "below" a second feature may refer to a first feature is in direct contact with a second feature, or a first feature is in indirect contact with a second feature via an intermediate medium. Further, a first feature is "above," "over" or "upside" a second feature may refer to a first feature is directly above or diagonally above a second feature, or may simply indicate that a first feature is higher horizontally than a second feature. A first feature "below," "under" or "downside" a second feature may refer to a first feature directly below or diagonally below a second feature, or may simply indicate that a first feature is lower horizontally than a second feature.

It should be noted that where an element is referred to as "fixed" or "configured" on another element, it may be directly on another element or there may be an intermediate element. When an element is considered to "connect to" another element, it may be directly connected to another element or there may be an intermediate element. The terms "vertical," "horizontal," "up," "down," "left," "right" and the like used herein are merely for illustrative purposes and are not intended to be the only implementation.

In view of the biological safety concerns of the medical metal material currently used in the clinical application due to the presence of nickel and cobalt, an implantable medical device, a method for manufacturing thereof, and a method for manufacturing a vascular stent are provided according to various embodiments of the present disclosure.

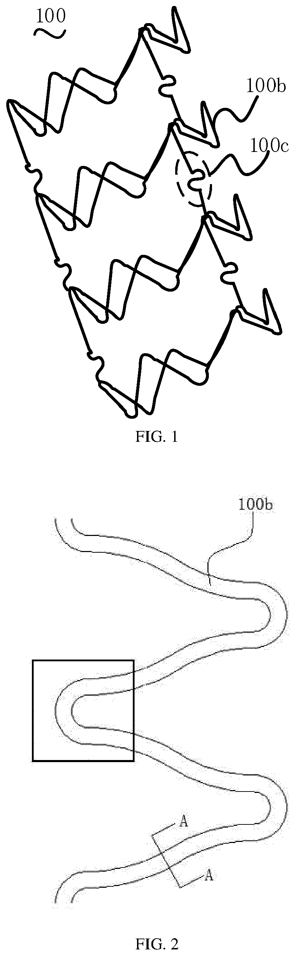

Embodiments of the present invention relate to an implantable medical device 100, which can be used as a medical device that can be implanted into in a patient's body, such as an orthopaedic implant or a vascular stent as shown in FIG. 1. When the implantable medical device 100 is used as a vascular stent, the implantable medical device 100 has a hollow tubular structure that is radially retractable or radially expandable. As an example, as shown in FIG. 1, the implantable medical device 100 used as a vascular stent includes a plurality of sets of supporting rods 100b and a plurality of connecting rods 100c. Each set of supporting rods 100b is configured in a wave-shaped structure connected end to end, and adjacent two sets of supporting rods 100b are connected by corresponding connecting rods 100c. For example, as shown in FIG. 1, adjacent two sets of supporting rods 100b can be connected by two connecting rods 100c.

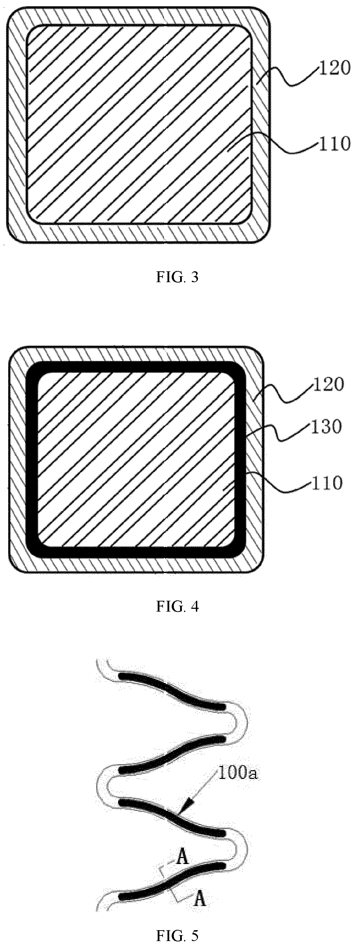

Referring to FIG. 3, FIG. 3 is a schematic cross-sectional view of the structure of an implantable medical device taken along line A-A in FIG. 2 according to one embodiment of the present disclosure. The implantable medical device 100 includes a metal substrate 110 and a cladding layer 120 provided on the metal substrate 110. The cladding layer 120 is a nickel-free and cobalt-free metal layer and is deposited on the metal substrate 110 by metal bonding. It should be noted that trace amount of nickel or cobalt is inevitably mixed in the process of manufacturing the above implantable medical device. Since the content of nickel and cobalt is generally within 0.001%, the content of nickel or cobalt is negligible. In addition, the cladding layer 120 according to an embodiment of the present disclosure is selected from medical metal materials having excellent and comprehensive mechanical properties such as strength, hardness, molding, impact toughness, fatigue resistance, excellent corrosion resistance, good biocompatibility, and excellent processing performance such as processing molding.

It should be understood that when the implantable medical device 100 is used as a vascular stent as shown in FIG. 1, the supporting rods 100b and the connecting rods 100c each includes the metal substrate 110 and the cladding layer 120. The cross-section of the metal substrate 110 can be round or polygonal with rounded corners, such as a regular quadrilateral structure with rounded corners as shown in FIG. 3. In addition, the structure of the cladding layer 120 can be substantially the same as that of the metal substrate 110.

As an example, the material of the metal substrate 110 can be selected from an iron-carbon alloy containing nickel (such as 316L stainless steel) or an iron-carbon alloy free of nickel; a cobalt-based alloy (such as L605 cobalt-chromium alloy or MP35N cobalt-chromium alloy); degradable iron and its alloy, degradable magnesium and its alloy, or degradable zinc and its alloy; a titanium alloy, a shape memory alloy, noble metal or pure metal tantalum, niobium, zirconium; or other medical metal materials. In one embodiment, the material of the metal substrate 110 can be selected from any one of iron-carbon alloys free of nickel, degradable iron and its alloy, degradable magnesium and its alloy, or degradable zinc and its alloy, so as to facilitate subsequent treatment of the implantable medical device 100 after removal from a patient's body.

As an example, the outer surface of the cladding layer 120 can be a rough surface or a smooth surface.

In the above implantable medical device 100, the cladding layer 120 is provided on the metal substrate 110 to prevent dissolution of sensitization and carcinogenic elements such as nickel and cobalt from the metal substrate 110. In addition, since the cladding layer 120 itself is a nickel-free and cobalt-free metal layer, there is no problem about dissolution of nickel and cobalt from the whole implantable medical device 100, thereby ensuring the biological safety of the implantable medical device 100 and further improving the biocompatibility of the implantable medical device 100. Furthermore, when the cladding layer is configured as the nickel-free and cobalt-free metal layer, the cladding layer itself can be deposited on the metal substrate by metal bonding, thereby effectively preventing the cladding layer from cracking or even peeling off. For example, when the implantable medical device is used as a vascular stent, the cladding layer will not be cracked or peeled off after the vascular stent is crimped and extremely dilated at least by 30% of the cold deformation amount. This can not only completely avoid dissolution of sensitization and oncogenic elements such as nickel and cobalt from the metal substrate, but also can improve corrosion resistance of the whole implantable medical device in a patient's body, thereby avoiding undesirable effects on the patient's health. For example, when the implantable medical device is used as a vascular stent, serious accidents such as thrombotic death due to peeling of the cladding layer can be avoided. It should be noted that if the metal substrate is directly provided as a nickel-free and cobalt-free metal substrate, it would be subjected to a series of procedures such as composition optimization design, experimental verification, ingot smelting, solution heat treatment, forging, rolling, annealing, tube blank processing, tube drawing, and stress relieving annealing, making the entire process have complicated procedures, a long processing period, a large investment, and high risks. However, in the embodiment of the present disclosure, the nickel-free and cobalt-free cladding layer 120 is provided on the metal substrate 110 by depositing, which is equivalent to modifying the surface of the metal substrate 110, thus it is not necessary to perform the above procedures, the processing procedures of the implantable medical device 100 can be simplified, the processing period of the implantable medical device 100 can be shortened, and the processing cost of the implantable medical device 100 can be reduced.

In some embodiments of the present disclosure, a density of the cladding layer 120 is greater than that of the metal substrate 110. Thus, the development performance of the implantable medical device 100 can be improved.

Optionally, the material of the cladding layer 120 can be selected from at least one of a metal element or a metal alloy. For example, the metal element can be Ir, Pt, Ru, Ta, W, or Au; and the metal alloy can be NiTi or/and a platinum-based binary alloy containing a noble metal which can include at least one of PtIr, PtPd, or PtRh.

The cladding layer 120 is selected from at least one of a metal element or a metal alloy, and according to the invention, , the cladding layer 120 contains hydroxyl groups. As an active oxygen, the hydroxyl groups can perform a reduction reaction and a complexation reaction with the metal of the cladding layer 120, activating the metal of the cladding layer 120 to be in an ionic state, and thus forming a more stable metal bond between the cladding layer 120 and the metal substrate 110, and further improving the bonding strength between the cladding layer 120 and the metal substrate 110.

Optionally, a mass fraction of hydroxyl groups can be less than or equal to 0.5%. For example, the mass fraction of hydroxyl groups of the cladding layer = relative atomic mass of hydroxyl groups × the number of hydroxyl groups/total molecular mass of the cladding layer (including an element or a compound) ×100%. For example, it can be 0.5%, 0.4%, or 0.3%, etc. Therefore, excessive addition of hydroxyl groups can be avoided while ensuring that the bonding strength between the cladding layer 120 and the metal substrate 110 can be effectively improved, thereby reducing the manufacturing cost of the implantable medical device 100. In one embodiment, the content of hydroxyl groups is less than or equal to 0.2%, such as 0.2%, 0.18%, 0.16%, 0.14%, or 0.12%, etc. In one embodiment, the content of hydroxyl groups is less than or equal to 0.1%, such as 0.1%, 0.08%, 0.06%, 0.04%, or 0.02%, etc.

Specifically, in some embodiments of the present disclosure, the thickness of the cladding layer 120 is less than or equal to 25 µm, such as 25 µm, 23 µm, 21 µm, 19 µm, or 17 µm, etc. Thus, the thickness of the implantable medical device 100 can be reduced while ensuring good visualization performance of the implantable medical device 100, thereby reducing the degree of irritation of the implantable medical device 100 to a patient. In one embodiment, the thickness of the cladding layer 120 is less than or equal to 15 µm, such as 15 µm, 14 µm, 13 µm, 12 µm, or 11 µm, etc. In one embodiment, the thickness of the cladding layer 120 is less than or equal to 10 µm, such as 10 µm, 9 µm, 8 µm, or 7 µm, etc. In one embodiment, the thickness of the cladding layer 120 is less than or equal to 6 µm, such as 6 µm, 5 µm, 4 µm, etc. Particularly, in one embodiment, the thickness of the cladding layer 120 is less than or equal to 3 µm, such as 3 µm, 2 µm, or 1 µm, etc.

According to the invention, as shown in FIG. 4, the implantable medical device 100 further includes at least one reinforcing layer 130 deposited between the metal substrate 110 and the cladding layer 120 by metal bonding. It should be noted that when the number of reinforcing layers 130 is plural, adjacent two reinforcing layers 130 can be deposited by metal bonding or other chemical bonding. The reinforcing layer 130 is configured to increase the bonding strength between the metal substrate 110 and the cladding layer 120, thereby effectively preventing the cladding layer 120 from cracking or even peeling off. In other embodiments, in application, the reinforcing layer 130 can also be deposited between metal substrate 110 and cladding layer 120 by other chemical bonding (such as covalent bonding). The material of the reinforcing layer 130 deposited by other chemical bonding (such as covalent bonding) can be the same as or different from that of the reinforcing layer 130 deposited between the metal substrate 110 and the cladding layer 120 by metal bonding.

It should be noted that the number of the reinforcing layers 130 is not specifically limited in the embodiment of the present disclosure, which can be set as one, two, three, etc.

As one example, a total thickness of the reinforcing layer 130 is less than that of the cladding layer 120. It should be understood that when the plurality of reinforcing layers 130 are deposited on metal substrate 110, the total thickness of the reinforcing layers 130 herein refers to the sum of the thicknesses of each reinforcing layer 130. When one reinforcing layer 130 is deposited on the metal substrate 110, the total thickness of the reinforcing layer 130 herein refers to the thickness of the reinforcing layer 130. Specifically, in some embodiments of the present disclosure, the thickness of the reinforcing layer 130 is less than 500 nm, such as 500 nm, 450 nm, 400 nm, or 350 nm, ect. Therefore, the thickness of the implantable medical device 100 can be reduced while ensuring that the bonding strength between the metal substrate 110 and the cladding layer 120 can be effectively improved, thereby reducing the degree of irritation of the implantable medical device 100 to a patient. In one embodiment, the thickness of the reinforcing layer 130 is less than or equal to 300 nm, such as 300 nm, 280 nm, 260 nm, 240 nm, or 220 nm, etc. In one embodiment, the thickness of the reinforcing layer 130 is less than or equal to 100 nm, such as 100 nm, 95 nm, 90 nm, or 85 nm, etc. In one embodiment, the thickness of the reinforcing layer 130 is 5 nm to 80 nm, such as 80 nm, 75 nm, 60 nm, 55 nm, 50 nm, 45 nm, 40 nm, 35 nm, 30 nm, 25 nm, 20 m, 15 nm, 10 nm, or 5 nm, etc.

Specifically, in some embodiments of the present disclosure, the material of the reinforcing layer 130 is selected from at least one of a metal element, a metal alloy, a metal oxide, or a metal nitride. For example, the metal element can be Ir, Pt, Ru, Ta, W, or Au. The metal oxide includes at least one of HfO₂, TiO₂, Ta₂O₅, ZnO, ZrO₂, MgO, SrTiOx, La₂O₃, or CeO₂. The metal nitride includes at least one of TiN or TaNx. The metal alloy can be NiTi or/and platinum-based binary alloys containing noble metals that can include at least one of PtIr, PtPd, or PtRh. In other embodiments, the material of the reinforcing layer 130 can also be the same as that of the cladding layer 120.

According to the invention the material of the reinforcing layer 130 is selected from a metal element, and a metal alloy, and the reinforcing layer 130 contains hydroxyl groups. As an active oxygen, the hydroxyl group can be perform a reduction reaction and a complexation reaction with the metal of the reinforcing layer 130, activating the metal of the reinforcing layer 130 to be in an ionic state. Therefore, a more stable metal bond can be formed between the two adjacent reinforcing layers 130, between the innermost reinforcing layer 130 and the metal substrate 110, and between the outermost reinforcing layer 130 and the cladding layer 120, thereby improving the bonding strength between the two adjacent reinforcing layers 130, between the innermost reinforcing layer 130 and the metal substrate 110, and between the outermost reinforcing layer 130 and the cladding layer 120.

Optionally, the mass fraction of hydroxyl groups is less than or equal to 0.5%, such as 0.5%, 0.4%, or 0.3%, etc. Thus, excessive addition of hydroxyl groups can be avoided while ensuring that the bonding strength between the two adjacent reinforcing layers 130, between the innermost reinforcing layer 130 and the metal substrate 110, and between the outermost reinforcing layer 130 and the cladding layer 120 can be effectively improved, thereby reducing the manufacturing cost of the implantable medical device 100. In one embodiment, the content of hydroxyl groups is less than or equal to 0.2%, such as 0.2%, 0.18%, 0.16%, 0.14%, or 0.12%, etc. In one embodiment, the content of hydroxyl groups is less than or equal to 0.1%, such as 0.1%, 0.08%, 0.06%, 0.04%, or 0.02%, etc.

As shown in FIG. 5, in some embodiments of the present disclosure, a drug-carrying slot 100a is provided on an outer wall of the implantable medical device 100. The drug-carrying slot 100a is configured to store drugs such as drugs for treating cardiovascular and cerebrovascular diseases, drugs for treating tumor diseases, drugs for treating intestinal diseases, drugs for treating urethral diseases, or the like, including but is not limited to at least one of paclitaxel, docetaxel, copper aspirinate, tacrolimus, hydroxy camptothecin, vinblastine, doxorubicin, rapamycin, or rapamycin derivatives. In other embodiments, at least one active drug layer can also be coated on an exterior of the cladding lay 120.

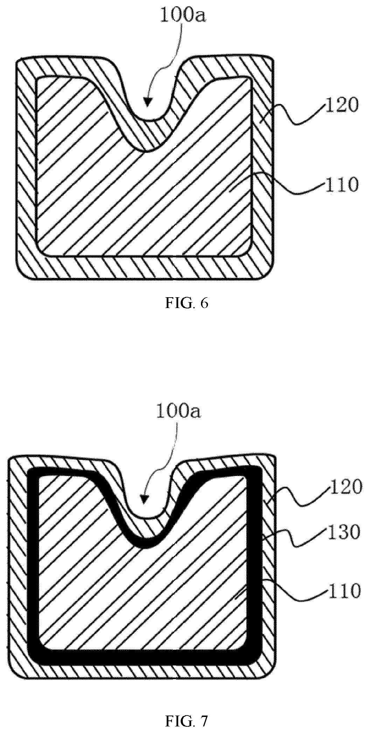

Specifically, in some embodiments of the present disclosure, the metal substrate 110 is provided with a groove, and a portion of the cladding layer 120 covering the groove is concaved toward the bottom of the groove to form the drug-carrying slot 100a, as shown in FIG. 6. Thus, in case that the thickness of the cladding layer 120 is not large enough, the depth of the drug-carrying slot 100a can be ensured. It should be noted that, as shown in FIG. 7, when the reinforcing layer 120 is provided between the metal substrate 110 and the cladding layer 120, the portions of the cladding layer 120 and the reinforcing layer 130 covering the groove are concaved toward the bottom of the groove to form the drug-carrying slot 100a.

In summary, the implantable medical device 100 according to the embodiment of the present disclosure can be used as an implantable medical stent, such as a vascular stent, which can effectively be removed, separated, or prevent dissolution of sensitization and oncogenic element such as nickel or cobalt, thereby improving biocompatibility of the implantable medical stent and extending population range suitable for the implantable medical stent. Furthermore, it can also solve the problem of cracking on the surface of the implantable medical stent, and ensure a good bonding force between the metal substrate 110 and a high-density metal layer (i.e., the cladding layer 120). In addition, it can improve the development performance of the implantable medical stent, thereby facilitating operation and clinical implementation by a physician.

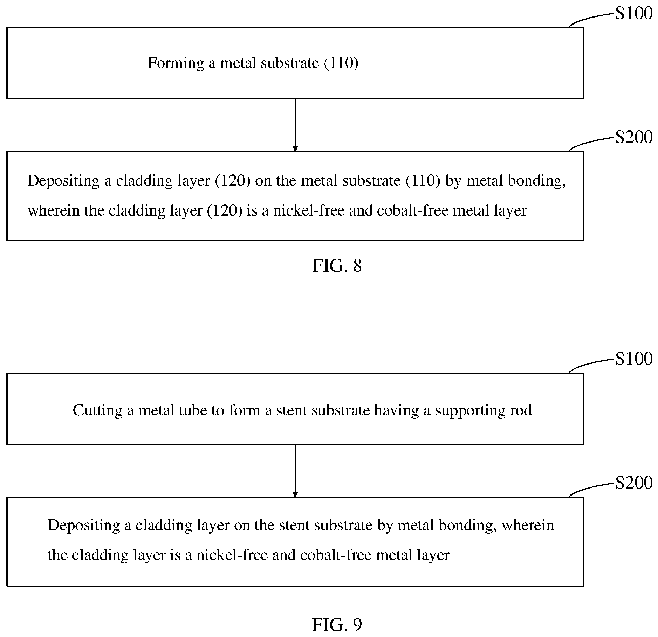

A method for manufacturing the above implantable medical device is further provided according to the invention. As shown in FIG. 8, the method includes:
Step S100, forming a metal substrate 110; and
Step S200, depositing a cladding layer 120 on the metal substrate 110 by metal bonding, wherein the cladding layer 120 is a nickel-free and cobalt-free metal layer.

In the above method for manufacturing the implantable medical device, the cladding layer 120 is provided on the metal substrate 110 to prevent dissolution of sensitization and carcinogenic element such as nickel and cobalt from the metal substrate 110. In addition, since the cladding layer 120 itself is a nickel-free and cobalt-free metal layer, there is dissolution of nickel and cobalt from the whole implantable medical device 100, thereby ensuring the biological safety of the implantable medical device 100 and further improving the biocompatibility of the implantable medical device 100. Moreover, the cladding layer 120 is deposited on the metal substrate 110, ensuring that the cladding layer 120 can be effectively prevented from cracking or even peeling off. For example, when the above implantable medical device is used as a vascular stent, the cladding layer 120 will not be cracked or peeled off after the vascular stent is crimped or dilated at least 30% of cold deformation amount. Thus, it can not only avoid dissolution of sensitization and carcinogenic elements such as nickel and cobalt from the metal substrate 110, but also can improve the overall corrosion resistance of the implantable medical device 100 in a patient's body, thereby avoiding undesirable effects on the patient's health. For example, when the implantable medical device is used as a vascular stent, serious accidents such as thrombotic death due to peeling of the cladding layer 120 can be avoided. It should be noted that, if the metal substrate is directly provided as a nickel-free and cobalt-free metal substrate, it would be required to be subjected to a series of procedures such as composition optimization design, experimental verification, ingot smelting, solution heat treatment, forging, rolling, annealing, tube blank processing, tube drawing, or stress relieving annealing, making the entire process have complicated procedures, a long processing period, a large investment, and high risks. However, in the implementation of the present disclosure, the nickel-free and cobalt-free cladding layer 120 is deposited on the metal substrate 110, which is equivalent to modifying the surface of the metal substrate 110, thus it is not necessary to perform the above procedures, the processing procedures of the implantable medical device 100 can be simplified, the processing period of the implantable medical device 100 can be shortened, and the processing cost of the implantable medical device 100 can be reduced.

As for step S100, specifically, the target material is cut to obtain the metal substrate 110. When the implantable medical device 100 is used as a vascular stent, the cut target material can be a hollow tubular structure that is radially retractable or radially expandable. In other embodiments, the metal substrate 110 can also be formed using a 3D printing method.

In application, after the target material is cut, the metal substrate 110 can be subjected to a series of treatments such as pickling, heating, or polishing, etc. Before the cladding layer 120 is deposited, the metal substrate 110 can be sequentially subjected to pretreatment, degreasing, pickling, activation by etching, or the like.

As for step S200, the deposition method can be a physical or chemical surface modification method, such as physical vapor deposition, thermal spraying, pulsed laser film deposition, magnetron sputtering deposition, evaporative plating, ion plating, chemical plating, electrochemical plating, chemical vapor deposition, anodic oxidation, ion implantation and deposition, or glow discharge plasma treatment, etc., or a combination thereof.

In application, in order to make the metal of the cladding layer 120 be in an activated ionic state, hydroxyl groups can be added to the cladding layer 120, which thus facilitates formation of a more stable metal bond between the cladding layer 120 and the metal substrate 110. The cladding layer 120 can be deposited on the metal substrate 110 by a solution plating method, such as physical vapor deposition, ion plating, electroless plating, or electrochemical plating, etc.

According to the invention , prior to performing the step S200, the method further includes a step S300, depositing at least one reinforcing layer 13 on the metal substrate 110 by metal bonding. It should be noted that when the number of the reinforcing layers 130 is plural, the adjacent two reinforcing layers 130 can also be deposited by metal bonding or other chemical bonding. The reinforcing layer 130 is configured to increase the bonding strength between the metal substrate 110 and the cladding layer 120, thereby effectively preventing the cladding layer 120 from cracking or even peeling off.

The deposition method can be a physical or chemical surface modification method, such as physical vapor deposition, thermal spraying, pulsed laser thin film deposition, magnetron sputtering deposition, evaporative plating, ion plating, chemical plating, electrochemical plating, chemical vapor deposition, anodic oxidation, ion implantation and deposition, glow discharge plasma treatment, etc., or a combination thereof.

In application, in order to make the metal of the reinforcing layer 130 be in an activated ionic state, hydroxyl groups can be added to the reinforcing layer 130, which facilitates formation of a more stable metal bond between the two adjacent reinforcing layers 130, between the innermost reinforcing layer 130 and the metal substrate 110, and between the outermost reinforcing layer 130 and the cladding layer 120. The reinforcing layer 130 can be deposited on the metal substrate 110 by a solution plating method, such as physical vapor deposition, ion plating, electroless plating, or electrochemical plating, etc.

In some embodiments of the present disclosure, prior to performing the step S200, the method further includes a step S400, forming a groove on the metal substrate 110 so that a drug-carrying slot 100a is formed on an exterior of the cladding layer 120 after the cladding layer 120 is provided on the metal substrate 110. For example, the drug-carrying slot 100a can be formed on the portion of the cladding layer 120 corresponding to the groove of the metal substrate 110.

The drug-carrying slot 100a is configured to store drugs such as drugs for treating cardiovascular and cerebrovascular diseases, drugs for treating tumor diseases, drugs for treating intestinal diseases, drugs for treating urethral diseases, or the like, including but is not limited to at least one of paclitaxel, docetaxel, copper aspirinate, tacrolimus, hydroxy camptothecin, vinblastine, doxorubicin, rapamycin, or rapamycin derivatives. In other embodiments, at least one active drug layer can also be coated on the exterior of the cladding layer 120.

A method for manufacturing a vascular stent is further described, as shown in FIG. 9. The method for manufacturing a vascular stent includes:
Step S100, cutting a metal tube to form a stent substrate having a supporting rod; and
Step S200, depositing a cladding layer on the stent substrate by metal bonding, wherein the cladding layer is a nickel-free and cobalt-free metal layer.

As an example, the supporting rod can be a wave-shaped rod.

In the above method for manufacturing the vascular stent, a cladding layer can be provided on the stent substrate to prevent dissolution of sensitization and carcinogenic elements such as nickel and cobalt from the stent substrate. In addition, since the cladding layer itself is a nickel-free and cobalt-free metal layer, there is no dissolution of nickel and cobalt from the whole vascular stent, thereby ensuring the biological safety of the vascular stent and further improving the biocompatibility of the vascular stent. Moreover, the cladding layer can be deposited on the stent substrate, thereby effectively preventing the cladding layer from cracking or even peeling off, and the cladding layer will not be cracked or peeled off after the vascular stent is crimped or dilated at least 30% of the cold deformation amount. Thus, it can completely avoid dissolution of sensitization and carcinogenic elements such as nickel and cobalt from the stent substrate, and improve corrosion resistance of the whole vascular stent in a patient's body, thereby avoiding undesirable effects on the patient's health. For example, it can avoid serious accidents such as thrombotic death due to peeling of the cladding layer. It should be noted that, if the metal substrate 110 is directly provided as a nickel-free and cobalt-free metal substrate, it would be required to be subjected to a series of procedures such as composition optimization design, experimental verification, ingot smelting, solution heat treatment, forging, rolling, annealing, tube blank processing, tube drawing, or stress relief annealing, making the entire process have complicated procedures, a long processing period, a large investment, and high risks. However, in the embodiment of the present disclosure, the nickel-free and cobalt-free cladding layer 120 is provided on the metal substrate 110 by depositing, which is equivalent to modifying the surface of the metal substrate 110, thus it is not necessary to perform the above procedures, the processing procedures of the implantable medical device 100 can be simplified, the processing period of the implantable medical device 100 can be shortened, and the processing cost of the implantable medical device 100 can be reduced.

As for step S100, after the metal tube is cut, a series of treatments such as pickling, heating, or polishing can be performed on the stent substrate. In addition, the stent substrate can be sequentially subjected to pretreatment, degreasing, pickling, activation by etching, or the like before the cladding layer is deposited.

As for step S200, the deposition method can be a physical or chemical surface modification method, such as physical vapor deposition, thermal spraying, pulsed laser film deposition, magnetron sputtering deposition, evaporative plating, ion plating, chemical plating, electrochemical plating, chemical vapor deposition, anodic oxidation, ion implantation and deposition, glow discharge plasma treatment, etc., or a combination thereof.

In application, in order to make the metal of the cladding layer be in an activated ionic state, hydroxyl groups can be added to the cladding layer, which facilitates formation of a more stable metal bond between the cladding layer and the stent substrate. The cladding layer can be deposited on the stent substrate by a solution coating method, such as physical vapor deposition, ion plating, electroless plating, or electrochemical plating, etc.

In some embodiments of the present disclosure, prior to performing the step S200, the method further includes a step S300, depositing at least one reinforcing layer 13 on the stent substrate by metal bonding. It should be noted that, when the number of reinforcing layers is plural, two adjacent reinforcing layers are also deposited by metal bonds or other chemical bonds. The reinforcing layer is configured to increase the bonding strength between the stent substrate and the cladding layer, thereby effectively preventing the cladding layer from cracking or even peeling off.

The deposition method can be a physical or chemical surface modification method, such as physical vapor deposition, thermal spraying, pulsed laser thin film deposition, magnetron sputtering deposition, evaporative plating, ion plating, chemical plating, electrochemical plating, chemical vapor deposition, anodic oxidation, ion implantation and deposition, glow discharge plasma treatment, etc., or a combination thereof.

In application, in order to make the metal of the reinforcing layer be in an activated ionic state, hydroxyl groups can be added to the reinforcing layer, which facilitates formation of a more stable metal bond between the two adjacent reinforcing layers, between the innermost reinforcing layer and the stent substrate, and between the outermost reinforcing layer and the cladding layer. The reinforcing layer can be deposited on the stent substrate by a solution plating method, such as a physical vapor deposition method, an ion plating method, an electroless plating method, an electrochemical plating method, or the like.

In some embodiments of the present disclosure, prior to performing the step S200, the method further includes a step S400, forming a groove on the stent substrate so that a drug-carrying slot is formed on the exterior of the cladding layer after the cladding layer is configured on the stent substrate. For example, a drug-carrying slot is formed on the portion of the cladding layer corresponding to the groove of the stent substrate.

The drug-carrying slot is configured to store drugs such as drugs for treating cardiovascular and cerebrovascular diseases, drugs for treating tumor diseases, drugs for treating intestinal diseases, drugs for treating urethral diseases, or the like, including but is not limited to at least one of paclitaxel, docetaxel, copper aspirinate, tacrolimus, hydroxy camptothecin, vinblastine, doxorubicin, rapamycin, or rapamycin derivatives. In other embodiments, at least one active drug layer can also be coated on the exterior of the cladding layer.

The present disclosure will be further described by specific Examples. Here, a vascular stent is used as an example in the following specific Examples.

The point at which the stress concentration of the vascular stent is maximum during extreme dilation is the position at which the cladding layer 120 is most likely to be cracked or peeled off after extreme dilation of the vascular stent, as shown in FIG. 2, where the reinforcing ring site of the vascular stent is indicated in the box. Therefore, whether the cladding layer 120 is cracked or peeled off after the reinforcing ring site is subjected to extreme dilation is mainly related to the bonding strength, i.e., bonding force, between the cladding layer 120 and the metal substrate 110. A high-resolution scanning electron microscope (SEM) visual observation method can be used to confirm whether the cladding layer 120 at the reinforcing ring site is cracked or peeled off after the stent is subjected to extreme dilation. In addition, corrosion resistance of the vascular stent can be tested according to YYT 0695-2008, Standard Test Method for Conducting Cyclic Potentiodynamic Polarization Measurements to Determine the Corrosion Susceptibility of Small Implant Devices (ASTM F 2129-06). As a Class III medical device, a vascular stent must be required to have excellent corrosion resistance and have a breakdown potential ≥600mV. It should be noted that, if the reinforcing ring site is found to have very fine microcracks under the high-resolution scanning electron microscope which are not easily to be visually detected after extreme dilation, it will be demonstrated that the galvanic cell reaction is formed between the cladding layer 120 and the metal substrate 110 due to different potential difference. If there is an obvious inflection point on the cyclic potentiodynamic polarization curve of corrosion resistance test, it would indicate that there is a breakdown potential, that is, pitting corrosion occurs. It should be noted that scanning electron microscopy and corrosion resistance test are non-destructive test methods, and in addition to these two methods, other destructive test methods can be used in other embodiments. For example, the bonding force between the cladding layer 120 and the metal substrate 110 can be tested by scratch test for measuring the bonding force of the coating according to the National Standard JB/T8554-1997.

In addition, extreme dilation of the vascular stent can be achieved according to a standard medical device implantation procedure as follows. Firstly, crimping the vascular stent onto a balloon, simulating through the human body in a phosphate buffer saline (PBS) solution at 37°C for three times, dilating to the nominal size of the vascular stent with an inflator, and continuing to increase the pressure until an extremely post-dilated size of the vascular stent is reached. On this basis, after reaching the extremely post-dilated size of the vascular stent, the vascular stent according to embodiments of the present disclosure is continued to be further post-dilated by balloon postdilation until the supporting rods of the vascular stent is straightened. It has been found that the actual post-dilated limit size of the vascular stent is about 25% larger than the nominal limit size. Thus, the validity of the present disclosure can be verified using this limit condition.

### Example 1

A vascular stent was provided in this Example. The material and thickness of the metal substrate 110 and the cladding layer 120 of the vascular stent are shown in Table 1. The vascular stent was manufactured by the following method. A magnesium alloy tubular member was cut to obtain a metal substrate 110, then the metal substrate 110 was subjected to pickling, heating, and polishing sequentially, and finally a layer of PtIr high-density metal, i.e., a cladding layer 120, with a thickness of 1 µm was deposited on the metal substrate 110 by electrochemical deposition. Specifically, the deposition process included liquid phase mass transfer, electrochemical reaction, and electrocrystallization. Prior to electrochemical deposition, the metal substrate 110 was subjected to pretreatment, degreasing, pickling, or activation by etching, and the cladding layer 120 was selected from a solution containing polar hydroxyl groups capable of forming a stable metal bond between metal interfaces to ensure good bonding force.

After extreme dilation of the vascular stent, the cladding layer 120 itself at the reinforcing ring site was tested to have no cracking or peeling off, and had a breakdown potential ≥600mV, showing that corrosion resistance test met the requirements. Moreover, the cladding layer was verified to have no cracking or peeling off by scratch test.

**Table 1**

| structural of the vascular stent | the metal substrate | the cladding layer |
|---|---|---|
| alloy type | magnesium alloy | PtIr |
| thickness | / | 1 µm |

### Examples 2-7

A vascular stent was respectively provided in Examples 2-7. The structure, the material and the thickness of each vascular stent were shown in Table 2.

**Table 2**

| | the metal substrate | the reinforcing layer and its thickness | the cladding layer and its thickness | the cladding layer condition after dilating of the reinforcing ring | corrosion resistance test | scratch test |
|---|---|---|---|---|---|---|
| Example 2 | ironbased alloy | Pt, 100nm | Pt, 3µm | no cracking or peeling off (see FIG.10(b)) | breakdown potential ≥600mV | the metal layer was not cracked or peeled off |
| Example 3 | titanium alloy | / | Ta, 2µm or W, 3µm | no cracking or peeling off (see FIG.10(c)) | breakdown potential ≥600mV | the metal layer was not cracked or peeled off |
| Example 4 | cobalt-based alloy | TiN, 200nm | Au, 10µm | no cracking or peeling off (see FIG.10(d)) | breakdown potential ≥600mV | the metal layer was not cracked or peeled off |
| Example 5 | shape memory alloy | TiN, 30nm and MgO, 70nm | Ir, 8µm | no cracking or peeling off (see FIG.10(e)) | breakdown potential ≥600mV | the metal layer was not cracked or peeled off |
| Example 6 | tantalum | / | W, 4µm | no cracking or peeling off (see FIG.10(f)) | breakdown potential ≥600mV | the metal layer was not cracked or peeled off |
| Example 7 | ironbased alloy | / | Au, 15µm | no cracking or peeling off (see FIG.10(g)) | breakdown potential ≥600mV | the metal layer was not cracked or peeled off |

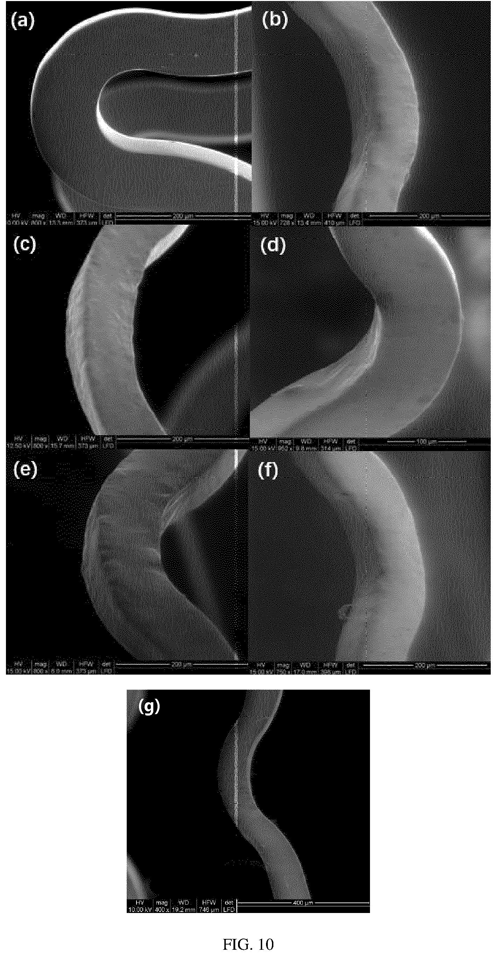

After extreme dilation, the cladding layer 120 itself at the reinforcing ring site of each vascular stent according to Example 2 to 7 was tested to have no cracking or peeling off, showing that the vascular stent each had excellent bonding strength; and had a breakdown potential ≥600mV, showing that corrosion resistance test met the requirements. Moreover, the cladding layer was verified to have no cracking or peeling off by scratch test. The structure of the reinforcing ring site of each vascular stent according to Example 2 to 7 prior to dilation was shown in FIG.10 (a).

### Example 8

A vascular stent with a drug-carrying slot 100a was provided in this Example. The material and thickness of the metal substrate 110 and the cladding layer 120 of the vascular stent were shown in Table 3.

**Table 3**

| structure of the vascular stent | the metal substrate | the reinforcing layer | the cladding layer |
|---|---|---|---|
| alloy type | cobalt-base alloy | HfO₂ | Au |
| thickness | / | 20nm | 1µm |

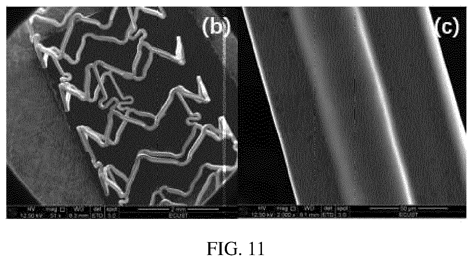

It can be seen from FIGS. 11(b) to 11(c) that, the drug-carrying slot 100a could run through the straight-line portion of the supporting rod 110b of the metal substrate (i.e., the area indicated as black in FIG. 5), and the cladding layer 120 corresponding to the extremely dilated straight-line portion of the vascular stent was not cracked or peeled off (see FIG. 11(b)). Moreover, the cladding layer 120 at the reinforcing ring site was not cracked or peeled off (see FIG. 11(c)), and had a breakdown potential ≥600 mV, showing that corrosion resistance test met the requirements. Moreover, the cladding layer 120 was verified to have no cracking or peeling off by scratch test.

## Claims

1. An implantable medical device, comprising a metal substrate (110) and a cladding layer (120); wherein the cladding layer (120) is a nickel-free and cobalt-free metal layer and is deposited on the metal substrate (110) by metal bonding;
wherein the implantable medical device (100) further comprises at least one reinforcing layer (130) deposited between the metal substrate (110) and the cladding layer (120) by metal bonding;
wherein a material of the reinforcing layer (130) is selected from at least one of a metal element and a metal alloy;
wherein the cladding layer (120) or/and the reinforcing layer (130) contain(s) hydroxyl groups.

2. The implantable medical device of claim 1, wherein a density of the cladding layer (120) is greater than a density of the metal substrate (110).

3. The implantable medical device of claim 1, wherein a thickness of the cladding layer (120) is less than or equal to 25 µm.

4. The implantable medical device of claim 1, wherein the material of the cladding layer (120) is selected from at least one of a metal element or a metal alloy; wherein the metal element is Ir, Pt, Ru, Ta, W or Au; and the metal alloy comprises at least one of PtIr, PtPd, or PtRh.

5. The implantable medical device of claim 1, wherein a total thickness of the reinforcing layer (130) is less than or equal to 500 nm.

6. The implantable medical device of claim 1, wherein the metal element is Ir, Pt, Ru, Ta, W or Au; the metal alloy comprises at least one of PtIr, PtPd, or PtRh.

7. A method for manufacturing the implantable medical device of any one of claims 1 to 6, comprising:
forming a metal substrate (110);
depositing at least one reinforcing layer (130) on the metal substrate (110) by metal bonding; and
depositing a cladding layer (120) on the metal substrate (110) by metal bonding, wherein the cladding layer (120) is a nickel-free and cobalt-free metal layer;
wherein a material of the reinforcing layer (130) is selected from at least one of a metal element and a metal alloy;
wherein the cladding layer (120) or/and the reinforcing layer (130) contain(s) hydroxyl groups.

8. The manufacturing method of claim 7, wherein the forming a metal substrate (110) comprises cutting a target material to obtain the metal substrate (110).

9. The manufacturing method of claim 7, wherein the cladding layer (120) or/and the reinforcing layer (130) are deposited by at least one of physical vapor deposition, thermal spraying, pulsed laser thin film deposition, magnetron sputtering deposition, evaporative plating, ion plating, electroless plating, electrochemical plating, chemical vapor deposition, anodic oxidation, ion implantation and deposition, or glow discharge plasma treatment.

## Patentansprüche

1. Implantierbare medizinische Vorrichtung, umfassend ein Metallsubstrat (110) und eine Umhüllungsschicht (120);
wobei die Umhüllungsschicht (120) eine nickelfreie und kobaltfreie Metallschicht ist und auf dem Metallsubstrat (110) durch Metallkleben abgeschieden wird;
wobei die implantierbare medizinische Vorrichtung (100) ferner mindestens eine Verstärkungsschicht (130), die zwischen dem Metallsubstrat (110) und der Umhüllungsschicht (120) durch Metallkleben abgeschieden wird, umfasst;
wobei ein Material der Verstärkungsschicht (130) aus mindestens einem von einem Metallelement und einer Metalllegierung ausgewählt ist;
wobei die Umhüllungsschicht (120) oder/und die Verstärkungsschicht (130) Hydroxylgruppen enthält/enthalten.

2. Implantierbare medizinische Vorrichtung nach Anspruch 1, wobei eine Dichte der Umhüllungsschicht (120) größer als eine Dichte des Metallsubstrats (110) ist.

3. Implantierbare medizinische Vorrichtung nach Anspruch 1, wobei eine Dicke der Umhüllungsschicht (120) kleiner oder gleich 25 µm ist.

4. Implantierbare medizinische Vorrichtung nach Anspruch 1, wobei das Material der Umhüllungsschicht (120) aus mindestens einem von einem Metallelement oder einer Metalllegierung ausgewählt ist; wobei das Metallelement Ir, Pt, Ru, Ta, W oder Au ist; und die Metalllegierung mindestens eines von Ptlr, PtPd oder PtRh umfasst.

5. Implantierbare medizinische Vorrichtung nach Anspruch 1, wobei eine Gesamtdicke der Verstärkungsschicht (130) kleiner als oder gleich 500 nm ist.

6. Implantierbare medizinische Vorrichtung nach Anspruch 1, wobei das Metallelement Ir, Pt, Ru, Ta, W oder Au ist; die Metalllegierung mindestens eines von Ptlr, PtPd oder PtRh umfasst.

7. Verfahren zum Herstellen der implantierbaren medizinischen Vorrichtung nach einem der Ansprüche 1 bis 6, umfassend:
Ausbilden eines Metallsubstrats (110);
Abscheiden mindestens einer Verstärkungsschicht (130) auf dem Metallsubstrat (110) durch Metallkleben; und
Abscheiden einer Umhüllungsschicht (120) auf dem Metallsubstrat (110) durch Metallkleben, wobei die Umhüllungsschicht (120) eine nickelfreie und kobaltfreie Metallschicht ist;
wobei ein Material der Verstärkungsschicht (130) aus mindestens einem von einem Metallelement und einer Metalllegierung ausgewählt ist;
wobei die Umhüllungsschicht (120) oder/und die Verstärkungsschicht (130) Hydroxylgruppen enthält/enthalten.

8. Herstellungsverfahren nach Anspruch 7, wobei das Ausbilden eines Metallsubstrats (110) ein Schneiden eines Zielmaterials, um das Metallsubstrat (110) zu erhalten, umfasst.

9. Herstellungsverfahren nach Anspruch 7, wobei die Umhüllungsschicht (120) oder/und die Verstärkungsschicht (130) durch mindestens eines von physikalischer Gasphasenabscheidung, thermischem Spritzen, gepulster Laserdünnfilmabscheidung, Magnetronsputterabscheidung, Verdampfungsplattierung, Ionenplattierung, stromloser Plattierung, elektrochemischer Plattierung, chemischer Gasphasenabscheidung, anodischer Oxidation, Ionenimplantation und -abscheidung oder Glimmentladungsplasmabehandlung abgeschieden werden.

## Revendications

1. Dispositif médical implantable, comprenant un substrat métallique (110) et une couche de revêtement (120) ;
dans lequel la couche de revêtement (120) est une couche métallique sans nickel et sans cobalt et est déposée sur le substrat métallique (110) par liaison métallique ;
dans lequel le dispositif médical implantable (100) comprend en outre au moins une couche de renfort (130) déposée entre le substrat métallique (110) et la couche de revêtement (120) par liaison métallique ;
dans lequel un matériau de la couche de renfort (130) est choisi parmi au moins l'un parmi un élément métallique et un alliage métallique ;
dans lequel la couche de revêtement (120) ou/et la couche de renfort (130) contiennent des groupes hydroxyle.

2. Dispositif médical implantable selon la revendication 1, dans lequel une densité de la couche de revêtement (120) est supérieure à une densité du substrat métallique (110).

3. Dispositif médical implantable selon la revendication 1, dans lequel une épaisseur de la couche de revêtement (120) est inférieure ou égale à 25 µm.

4. Dispositif médical implantable selon la revendication 1, dans lequel le matériau de la couche de revêtement (120) est choisi parmi au moins un élément métallique ou un alliage métallique ; dans lequel l'élément métallique est Ir, Pt, Ru, Ta, W ou Au ; et l'alliage métallique comprend au moins l'un parmi Ptlr, PtPd ou PtRh.

5. Dispositif médical implantable selon la revendication 1, dans lequel une épaisseur totale de la couche de renfort (130) est inférieure ou égale à 500 nm.

6. Dispositif médical implantable selon la revendication 1, dans lequel l'élément métallique est Ir, Pt, Ru, Ta, W ou Au ; l'alliage métallique comprend au moins l'un parmi PtIr, PtPd ou PtRh.

7. Procédé de fabrication du dispositif médical implantable selon l'une quelconque des revendications 1 à 6, comprenant :
la formation d'un substrat métallique (110) ;
le dépôt d'au moins une couche de renfort (130) sur le substrat métallique (110) par liaison métallique ; et
le dépôt d'une couche de revêtement (120) sur le substrat métallique (110) par liaison métallique, dans lequel la couche de revêtement (120) est une couche métallique sans nickel et sans cobalt ;
dans lequel un matériau de la couche de renfort (130) est choisi parmi au moins l'un parmi un élément métallique et un alliage métallique ;
dans lequel la couche de revêtement (120) ou/et la couche de renfort (130) contiennent des groupes hydroxyle.

8. Procédé de fabrication selon la revendication 7, dans lequel la formation d'un substrat métallique (110) comprend la découpe d'un matériau cible pour obtenir le substrat métallique (110).

9. Procédé de fabrication selon la revendication 7, dans lequel la couche de revêtement (120) ou/et la couche de renfort (130) sont déposées par au moins l'un parmi le dépôt physique en phase vapeur, la pulvérisation thermique, le dépôt de couche mince au laser pulsé, le dépôt par pulvérisation magnétron, le placage par évaporation, le placage ionique, le placage autocatalytique, le placage électrochimique placage, le dépôt chimique en phase vapeur, l'oxydation anodique, l'implantation et le dépôt d'ions, ou le traitement au plasma à décharge luminescente.
